(19) Europäisches Patentamt / European Patent Office / Office européen des brevets

(11) **EP 4 707 376 A1**

(12) **EUROPEAN PATENT APPLICATION**
published in accordance with Art. 153(4) EPC

(43) Date of publication:
**11.03.2026 Bulletin 2026/11**

(21) Application number: **24826249.5**

(22) Date of filing: **19.06.2024**

(51) International Patent Classification (IPC):
*C12N 1/02* (2006.01)     *C12N 1/20* (2026.01)
*C12R 1/125* (2006.01)

(86) International application number:
**PCT/KR2024/008471**

(87) International publication number:
**WO 2024/262934 (26.12.2024 Gazette 2024/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.06.2023 KR 20230078261**

(71) Applicant: **CJ Cheiljedang Corporation**
**Seoul 04560 (KR)**

(72) Inventors:
  • **LEE, Sungkyun**
    **Seoul 04560 (KR)**
  • **PARK, Jung Won**
    **Seoul 04560 (KR)**
  • **KIM, Taekbeom**
    **Seoul 04560 (KR)**
  • **LEE, Hyo Hyoung**
    **Seoul 04560 (KR)**
  • **KIM, Young Kyung**
    **Seoul 04560 (KR)**
  • **KIM, Yu Shin**
    **Seoul 04560 (KR)**
  • **KIM, Minhoe**
    **Seoul 04560 (KR)**

(74) Representative: **Meissner Bolte Partnerschaft mbB**
**Patentanwälte Rechtsanwälte**
**Postfach 86 06 24**
**81633 München (DE)**

(54) **METHOD FOR SEPARATING AND REMOVING MICROBIAL CELLS FROM FERMENTATION SOLUTION**

(57)     Provided are a method of separating and removing *Bacillus subtilis* cells from a *Bacillus subtilis* fermentation broth, and a composition for removing *Bacillus subtilis* cells from the *Bacillus subtilis* fermentation broth.

[FIG. 1]

**Description**

**[Technical Field]**

The present disclosure relates to a method of separating and removing *Bacillus subtilis* cells from a *Bacillus subtilis* fermentation broth, and a composition for removing *Bacillus subtilis* cells from the *Bacillus subtilis* fermentation broth.

**[Background Art]**

Generally, fermentation processes using microorganisms are mainly used for the mass production of target products such as enzymes, amino acids, etc. However, the production of target products through microbial fermentation processes has a problem of requiring significant purification costs due to the presence of various byproducts, as impurities, comprising microbial cells and organic acids along with the target products in the fermentation broth.

Meanwhile, *Bacillus subtilis* cells are known to be difficult to separate and remove due to their small size and high viscosity. To remove microbial cells, therefore, methods such as dilution or heating of fermentation broth, use of large amounts of filter aids, etc. are used. However, there are problems in that these methods may inactivate target substances, proteins (enzymes), or may leave the same in the process, thereby reducing the efficiency of the process of separating and removing the microbial cells and subsequent processes. For example, microbial cells that remain unremoved may reduce flux and membrane life in MF/UF membrane processes, thereby generating shear stress during the process. Ultimately, there is a problem of reducing the recovery rate of target proteins (enzymes).

In this regard, KR 10-2013-0022059A discloses, as a method oseparating microbial cells from a fermentation broth, centrifugation, filter presses, pressure filters, diatomaceous earth filters, rotary vacuum filters, membrane separators, and flocculation and flotation methods, etc. However, this prior art does not mention a method of separating and removing *Bacillus subtilis* cells from a *Bacillus subtilis* fermentation broth using calcium salts and phosphates.

Accordingly, research is still needed to separate and remove *Bacillus subtilis* cells from the *Bacillus subtilis* fermentation broth using simpler and more economical processes.

**[Disclosure]**

**[Technical Problem]**

An object of the present disclosure is to provide a method of separating and removing *Bacillus subtilis* cells from a *Bacillus subtilis* fermentation broth, the method comprising the steps of preparing the *Bacillus subtilis* fermentation broth; adding calcium salts and phosphates to the *Bacillus subtilis* fermentation broth; and removing the *Bacillus subtilis* cells from the fermentation broth to which the calcium salts and phosphates have been added.

Another object of the present disclosure is to provide a composition for removing *Bacillus subtilis* cells from a *Bacillus subtilis* fermentation broth, the composition comprising calcium salts and phosphates.

**[Technical Solution]**

There is provided a method of separating and removing *Bacillus subtilis* cells from a *Bacillus subtilis* fermentation broth, the method comprising the steps of preparing the *Bacillus subtilis* fermentation broth; adding calcium salts and phosphates to the *Bacillus subtilis* fermentation broth; and removing the *Bacillus subtilis* cells from the fermentation broth to which the calcium salts and phosphates have been added.

There is also provided a composition for removing *Bacillus subtilis* cells from a *Bacillus subtilis* fermentation broth, the composition comprising calcium salts and phosphates.

**[Advantageous Effects]**

A method of separating and removing *Bacillus subtilis* cells from a *Bacillus subtilis* fermentation broth of the present disclosure has an excellent effect of increasing the process yield and economic feasibility, since calcium chloride and monosodium phosphate are used to effectively remove microbial cells and impurities generated as fermentation byproducts in the fermentation broth during a fermentation process using *Bacillus subtilis* by a simple process without comprising a step of diluting the *Bacillus subtilis* fermentation broth, and accordingly, the method may be usefully applied in the industrial production of a target product.

**[Brief Description of the Drawings]**

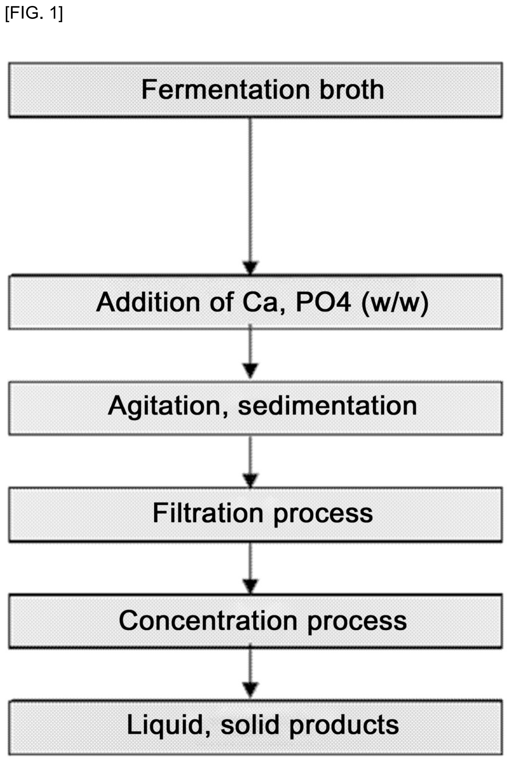

FIG. 1 is a flow chart illustrating a method of separating and removing *Bacillus subtilis* cells from a *Bacillus subtilis* fermentation broth according to one embodiment of the present disclosure.

**[Brief Description of the Drawing]**

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

As used in the specification and appended claims of the present disclosure, the singular forms ("a", "an", and "the") comprise plural referents unless the context clearly states otherwise. Unless the context indicates otherwise, singular terms shall comprise the plural and plural terms shall comprise the singular. As used in the specification and appended claims of the present disclosure, unless stated otherwise, the use of "or" may be used to comprise "and/or".

As used herein, the term "about" may be presented before a particular numerical value. The term "about" used herein comprises not only the exact number recited after the term, but also a range that is near or close to that number. Considering the context in which the number is presented, it may be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third," "i), ii), iii)," or "(a), (b), (c), (d)" are used to distinguish similar constitutions, and these terms do not mean that the constitutions are performed continually or sequentially. For example, when the terms are used in reference to steps of a method, use, or assay, there may be no time interval between these steps, or they may be performed concurrently, or may be performed several seconds, several minutes, several hours, several days, or several months apart.

As used herein, the term "comprising" means the presence of features, steps or components recited after the term, and does not exclude the presence or addition of one or more features, steps or components. The components or features recited after the term "comprising" herein may be essential or mandatory. However, in some embodiments, the term may further comprise any other or non-essential components or features.

An aspect of the present disclosure provides a method of separating and removing *Bacillus subtilis* cells from a *Bacillus subtilis* fermentation broth, the method comprising the steps of preparing the *Bacillus subtilis* fermentation broth; adding calcium salts and phosphates to the *Bacillus subtilis* fermentation broth; and removing the *Bacillus subtilis* cells from the fermentation broth to which the calcium salts and phosphates have been added.

In a specific embodiment, the calcium salts may be calcium chloride ($CaCl_2$).

In a specific embodiment, the phosphates may be selected from the group consisting of sodium dihydrogen phosphate ($NaH_2PO_4$), phosphoric acid ($H_3PO_4$), disodium hydrogen phosphate ($Na_2HPO_4$), potassium dihydrogen phosphate ($KH_2PO_4$), dipotassium hydrogen phosphate ($K_2HPO_4$), ammonium dihydrogen phosphate ($NH_4H_2PO_4$), diammonium hydrogen phosphate ($(NH_4)_2HPO_4$), and combinations thereof, and specifically, sodium dihydrogen phosphate ($NaH_2PO_4$), but are not limited thereto.

In a specific embodiment, the method of separating and removing *Bacillus subtilis* cells from the *Bacillus subtilis* fermentation broth of the present disclosure may comprise the steps of preparing the *Bacillus subtilis* fermentation broth; adding calcium chloride ($CaCl_2$) and sodium dihydrogen phosphate ($NaH_2PO_4$) to the *Bacillus subtilis* fermentation broth; and removing the *Bacillus subtilis* cells from the fermentation broth to which the calcium chloride and sodium dihydrogen phosphate have been added, wherein the calcium chloride is comprised in an amount of 0.5 parts by weight to 0.75 parts by weight, based on the total 100 parts by weight of the composition, and the sodium dihydrogen phosphate is comprised in an amount of 0.9 parts by weight to 1.5 parts by weight, based on the total 100 parts by weight of the composition, and the calcium chloride and sodium dihydrogen phosphate are comprised in a mixing ratio of 1 : 1.6 to 1 : 2.

Generally, fermentation processes using microorganisms are mainly used for the mass production of target products such as enzymes, amino acids, etc. However, the production of target products through microbial fermentation processes has a problem of requiring significant purification costs due to the presence of various byproducts, as impurities, comprising microbial cells and organic acids along with the target products in the fermentation broth.

Particularly, in fermentation process using *Bacillus subtilis, Bacillus subtilis* cells are known to be difficult to separate and remove due to their small size and high viscosity. To remove microbial cells, therefore, methods such as dilution or heating of fermentation broth, use of large amounts of filter aids, etc. are used. However, there are problems in that these methods may inactivate target substances, proteins (enzymes), or may leave the same in the process, thereby reducing the efficiency of the process of separating and removing the microbial cells and subsequent processes. For example, microbial cells that remain unremoved may reduce flux and membrane life in MF/UF membrane processes, thereby generating shear stress during the process. Ultimately, there is a problem of reducing the recovery rate of target proteins (enzymes).

The method of separating and removing *Bacillus subtilis* cells from the *Bacillus subtilis* fermentation broth of the present disclosure has an excellent effect of increasing the process yield and economic feasibility, since calcium chloride and sodium dihydrogen phosphate are used to effectively remove microbial cells and impurities generated as fermentation byproducts in the fermentation broth during a fermentation process using *Bacillus subtilis* by a simple process.

As used herein, the term "fermentation" refers to a process whereby bacteria break down organic materials using their own enzymes, excluding putrefaction. Fermentation and putrefaction proceed by similar processes. However, when useful substances are produced as a result of decomposition, it is called fermentation, and when bad smells or harmful substances are produced, it is called putrefaction.

In the present disclosure, the method of obtaining the *Bacillus subtilis* fermentation broth is not particularly limited, and the fermentation product may be obtained according to a method commonly used in the art or similar art.

As used herein, the term "fermentation broth" comprises not only a fermented material itself, but also all types of materials comprising the fermentation product generated from the strain, such as a culture medium of a strain in which the strain and the culture coexist, a fermentation product obtained by filtering the strain from the culture medium, a fermentation product obtained by sterilizing the strain from the culture medium and filtering the same, an extract obtained by extracting the fermentation product or the culture medium comprising the same, a dilution obtained by diluting the fermentation product or the extract thereof, a concentrate, a dry product obtained by drying the fermentation product or the extract thereof, a lysate obtained by collecting and disrupting microbial cells of the strain, etc.

In a specific embodiment, the calcium chloride may be comprised in an amount of 0.1 part by weight to 2 parts by weight, 0.2 parts by weight to 2.0 parts by weight, 0.3 parts by weight to 2 parts by weight, 0.4 parts by weight to 2 parts by weight, 0.5 parts by weight to 2.0 parts by weight, 0.5 parts by weight to 1.5 parts by weight, or 0.5 parts by weight to 0.75 parts by weight, based on the total 100 parts by weight of the composition, but is not limited thereto.

The sodium dihydrogen phosphate may be comprised in an amount of 0.5 parts by weight to 4 parts by weight, 0.6 parts by weight to 4 parts by weight, 0.7 parts by weight to 4 parts by weight, 0.8 parts by weight to 4 parts by weight, 0.8 parts by weight to 3 parts by weight, or 0.9 parts by weight to 1.5 parts by weight, based on the total 100 parts by weight of the composition, but is not limited thereto.

In a specific embodiment, the calcium chloride and sodium dihydrogen phosphate may be comprised in a mixing ratio of 1 : 1 to 1 : 3, 1 : 1.1 to 1 : 2.9, 1 : 1.2 to 1 : 2.8, 1 : 1.3 to 1 : 2.7, 1 : 1.4 to 1 : 2.6, 1 : 1.5 to 1 : 2.5 or 1 : 1.6 to 1 : 2, but is not limited thereto.

By comprising the calcium chloride and sodium dihydrogen phosphate in the above range and/or the mixing ratio, it is possible to effectively remove microbial cells and impurities generated as fermentation byproducts in the fermentation broth during the fermentation process using *Bacillus subtilis* by the simple process without comprising a step of diluting the *Bacillus subtilis* fermentation broth, and therefore, there is an excellent effect of increasing the process yield and economic feasibility. In particular, since the step of diluting the *Bacillus subtilis* fermentation broth is not comprised, the addition amounts of the calcium chloride and sodium dihydrogen phosphate are minimized while achieving the optimal amounts capable of exhibiting the maximum effect of separating and removing microbial cells, which is effective in terms of process cost and process time, as compared to the existing process of separating and removing microbial cells comprising the dilution step.

With regard to the method of separating and removing *Bacillus subtilis* cells from the *Bacillus subtilis* fermentation broth of the present disclosure, the fermentation broth from which the *Bacillus subtilis* cells are removed may have a *Bacillus subtilis* cell removal rate of 65% or more, 66% or more, 67% or more, 68% or more, 69% or more, 70% or more, 75% or more, 80% or more, 85% or more, 90% or more, 95% or more, 96% or more, 97% or more, 98% or more, or 99% or more.

The method of separating and removing *Bacillus subtilis* cells from the *Bacillus subtilis* fermentation broth of the present disclosure may not comprise the step of diluting the *Bacillus subtilis* fermentation broth.

Generally, upon using additives for flocculating microbial cells comprised in a fermentation broth, the fermentation broth is used after being diluted 2 times to 10 times, and thus there are problems that the amount of the solution to be treated is increased, the amount of use of a flocculating agent is increased, and the process time is increased.

The method of separating and removing *Bacillus subtilis* cells from the *Bacillus subtilis* fermentation broth of the present disclosure does not comprise the step of diluting the *Bacillus subtilis* fermentation broth, thereby shortening the process time and reducing losses in subsequent processes. Specifically, since the method does not comprise the dilution step, the amount of the solution to be treated is reduced 2 times to 10 times, thereby reducing the amount of use of the flocculating agent, and reducing the amount of use of auxiliary materials, such as filter aids, filters, and electricity, which are used in separating and removing microbial cells and impurities after flocculating, and shortening the filtration and concentration process time, and accordingly, there is an excellent effect of significantly reducing the manufacturing costs.

In a specific embodiment, the *Bacillus subtilis* fermentation broth may be prepared by culturing *Bacillus subtilis* in a medium, but is not limited thereto.

As used herein, the term "culturing" refers to growing the strain of the present disclosure under appropriately adjusted environmental conditions. The culture procedure of the present disclosure may be performed according to appropriate media or culture conditions known in the art. Such a culture procedure may be easily adjusted for use by a person skilled in the art according to the selected strain. Specifically, the culturing may be in a batch type, a continuous type, and/or a fed-batch type, but is not limited thereto.

As used herein, the term "medium" refers to a mixture comprising, as main ingredients, nutrient materials required for culturing the microorganism of the present disclosure, wherein the medium supplies nutrient materials comprising water essential for survival and growth, growth factors, etc. Specifically, as for the media and other culture conditions used for culturing the strain of the present disclosure, any medium that is used for usual culture of microorganisms may be used without particular limitation. However, the microorganisms of the present disclosure may be cultured under aerobic conditions in a common medium comprising appropriate carbon sources, nitrogen sources, phosphorus sources, inorganic compounds, amino acids, and/or vitamins, while controlling the temperature, pH, etc.

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fish or decomposition products thereof, defatted soybean cake or decomposition products thereof, etc. may be used. These nitrogen sources may be used alone or in a combination of two or more thereof, but are not limited thereto.

The phosphate sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These components or precursors may be added to the medium in a batch or continuous manner. However, the present disclosure is not limited thereto.

Further, pH of the medium may be adjusted by adding compounds, such as ammonium hydroxide, potassium hydroxide, ammonia, phosphoric acid, sulfuric acid, etc., to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-comprising gas may be injected into the medium to maintain the aerobic state of the medium, or no gas may be injected, or nitrogen, hydrogen, or carbon dioxide gas may be injected to maintain the anaerobic or non-aerobic state, but is not limited thereto.

In the culture of the present disclosure, the culture temperature may be maintained at 27°C to 37°C, specifically, at 30°C to 33°C, and the culture may be performed for about 20 hours to 120 hours, but is not limited thereto.

As used herein, the term "culture" refers to a culture liquid obtained by culturing a specific microorganism in a culture medium, a concentrated culture liquid, a dry product of the culture liquid, a culture filtrate, a concentrated culture filtrate, or a dry product of the culture filtrate, and the culture liquid refers to those comprising the specific microorganism, and the culture filtrate refers to those substantially not comprising the specific microorganism (which means that the microorganism to be separated by filtration, etc. are substantially excluded, which does not mean that the microorganism is completely excluded from the filtrate). The formulation of the culture is not limited, but it may be, for example, a liquid, an emulsion, or a solid. Specifically, with respect to the objects of the present disclosure, the culture may comprise the target product.

With regard to the method of the present disclosure, the culturing of the microorganism may be performed using any culturing conditions and culturing method known in the art. Such culturing procedure may be easily adjusted for use by a person skilled in the art according to the selected strain.

In a specific embodiment, before the step of removing the *Bacillus subtilis* cells from the fermentation broth to which the calcium chloride and sodium dihydrogen phosphate have been added, the method may further comprise the step of adjusting the pH of the *Bacillus subtilis* fermentation broth to 6.5 to 7.5, 6.6 to 7.4, 6.7 to 7.3, 6.8 to 7.2, 6.9 to 7.1 or 6.95 to 7.05.

Another aspect of the present disclosure provides a composition for removing *Bacillus subtilis* cells from a *Bacillus subtilis* fermentation broth, the composition comprising calcium chloride and sodium dihydrogen phosphate.

The calcium chloride, sodium dihydrogen phosphate, and *Bacillus subtilis* fermentation broth are as described above.

In a specific embodiment, the composition is a composition for removing *Bacillus subtilis* cells from a *Bacillus subtilis* fermentation broth, the composition comprising calcium chloride and sodium dihydrogen phosphate, wherein the calcium chloride is comprised in an amount of 0.5 parts by weight to 0.75 parts by weight, based on the total 100 parts by weight of the composition, the sodium dihydrogen phosphate is comprised in an amount of 0.9 parts by weight to 1.5 parts by weight, based on the total 100 parts by weight of the composition, and the calcium chloride and sodium dihydrogen phosphate are comprised in a mixing ratio of 1 : 1.6 to 1 : 2.

In a specific embodiment, the calcium chloride may be comprised in an amount of 0.1 part by weight to 2 parts by weight, 0.2 parts by weight to 2.0 parts by weight, 0.3 parts by weight to 2 parts by weight, 0.4 parts by weight to 2 parts by weight, 0.5 parts by weight to 2.0 parts by weight, 0.5 parts by weight to 1.5 parts by weight, or 0.5 parts by weight to 0.75 parts by weight, based on the total 100 parts by weight of the composition, but is not limited thereto.

The sodium dihydrogen phosphate may be comprised in an amount of 0.5 parts by weight to 4 parts by weight, 0.6 parts by weight to 4 parts by weight, 0.7 parts by weight to 4 parts by weight, 0.8 parts by weight to 4 parts by weight, 0.8 parts by weight to 3 parts by weight, or 0.9 parts by weight to 1.5 parts by weight, based on the total 100 parts by weight of the composition, but is not limited thereto.

In a specific embodiment, the calcium chloride and sodium dihydrogen phosphate may be comprised in a mixing ratio of 1 : 1 to 1 : 3, 1 : 1.1 to 1 : 2.9, 1 : 1.2 to 1 : 2.8, 1 : 1.3 to 1 : 2.7, 1 : 1.4 to 1 : 2.6, 1 : 1.5 to 1 : 2.5 or 1 : 1.6 to 1 : 2, but is not limited thereto.

By comprising the calcium chloride and sodium dihydrogen phosphate in the above range and/or the mixing ratio, it is possible to effectively remove microbial cells and impurities generated as fermentation byproducts in the fermentation broth during the fermentation process using *Bacillus subtilis* by the simple process without comprising a step of diluting the *Bacillus subtilis* fermentation broth, and therefore, there is an excellent effect of increasing the process yield and economic feasibility. In particular, since the step of diluting the *Bacillus subtilis* fermentation broth is not comprised, the addition amounts of the calcium chloride and sodium dihydrogen phosphate are minimized while achieving the optimal amounts capable of exhibiting the maximum effect of separating and removing microbial cells, which is effective in terms of process cost and process time, as compared to the existing process of separating and removing microbial cells comprising the dilution step.

In a specific embodiment, pH of the composition for removing *Bacillus subtilis* cells from the *Bacillus subtilis* fermentation broth of the present disclosure may be 6.5 to 7.5, 6.6 to 7.4, 6.7 to 7.3, 6.8 to 7.2, 6.9 to 7.1, or 6.95 to 7.05.

**[Brief Description of the Drawing]**

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

**Example. Evaluation of microbial cell removal rates of $NaH_2PO_4$ and $CaCl_2$**

It was intended to evaluate the microbial cell removal rates of $NaH_2PO_4$ and $CaCl_2$.

<Experimental Method>

$CaCl_2$ (Jiangsu Kolod Food Ingredients Co., Ltd.) and $NaH_2PO_4$ (Jiangsu Runpu Food Technology Co., Ltd.) were used. The addition amount of $CaCl_2$ was 0.5% to 0.75% of the weight of a fermentation broth, and the addition amount of $NaH_2PO_4$ was 0.9% to 1.5% of the weight of the fermentation broth. In addition, the addition ratio of $CaCl_2$:$NaH_2PO_4$ ranged from 1:1.6 to 1:2.0. A centrifuge (Shanghai Anting Science Instrument Factory, TDL-60B) used to confirm the flocculation effect and to analyze the physical properties was operated under conditions of 3,000 rpm to 4,000 rpm for 5 minutes to 15 minutes.

Fermentation of a *Bacillus subtilis* strain (KCTC 18039P, KR 100411238 B1) was conducted using a fed-batch culture. Dissolved oxygen (DO) was used as an indicator, and a feeding medium was fed when DO increased. In this regard, the composition of the main fermentation medium consisted of lactose, corn steep liquor, calcium chloride, sodium dihydrogen phosphate, ammonium sulfate, and magnesium sulfate. The feeding medium consisted of lactose, manganese sulfate, iron sulfate, and zinc sulfate. Meanwhile, as for the fermentation conditions, the culture temperature was maintained at 32°C while adjusting the agitation speed to avoid DO limitations.

In detail, without a dilution step, a flocculating agent was added to a stock solution of the *Bacillus subtilis* fermentation broth at a ratio of 1:1.6, 1:1.8, or 1:2.0. After gentle agitation for approximately 1 hour and sedimentation, microbial cells were removed using centrifugation or filtration. The microbial cell removal rate was calculated by analyzing the physical properties of the solution before and after removal of the microbial cells. The weight (DCW, Dry Cell Weight) of the microbial cells was calculated by measuring total solids (TS) and total dissolved solids (TDS). The method of measuring the physical properties of the liquid, and the calculation formula are as follows:

TS: 2 g to 3 g of the sample was accurately weighed with an accuracy of 0.001 g in a weighing bottle, placed in an oven, and dried at 105°C for 3 hours, and then the sample was placed in a desiccator, and cooled to room temperature (30 minutes), and dried. The calculation formula is as follows:

A: Weight of the weighing bottle (g), B: Weight of the sample and the weighing bottle (g), C: Weight of the weighing bottle and the dried weight (g)

$$TS = 1-(B-C)/(B-A)*100\%$$

TDS: The sample was placed in a centrifuge, centrifuged at 4000 rpm for 5 minutes (when centrifugation was not possible, it was filtered with 0.45 um), and then 2 g to 3 g of the centrifuged supernatant was accurately weighed with an accuracy of 0.001 g in a weighing bottle, placed in an oven, dried at 105°C for 3 hours, and then placed in a desiccator, cooled to room temperature (30 minutes), and dried. The calculation formula is as follows.

A: Weight of the weighing bottle (g), B: Weight of the sample and weighing bottle (g), C: Weight of the weighing bottle and the dried weight (g)

$$TDS = 1-(B-C)/(B-A)*100\%$$

PCV: the sample was placed in a centrifuge using a centrifuge tube and centrifuged at 3,000 rpm for 15 minutes. The calculation formula is as follows:

A: Volume of the sample before centrifugation, B: Volume of the supernatant after centrifugation

$$PCV = (A-B)/A*100\%$$

DCW: The weight of microbial cells was calculated using the measured TS and TDS. The calculation formula is as follows:

$$DCW = TS-(TDS*((100-TS)/(100-TDS)))$$

**<Comparative Example>**

With regard to evaluation of a control group, **$NaH_2PO_4$** and **$CaCl_2$** were not added to the *Bacillus subtilis* fermentation broth, and the pH was not adjusted, and the microbial cell removal rate was evaluated through centrifugation or filtration processes. The results are shown in Table 1 below.

[Table 1]

| Evaluation conditions | | | | | | Fermentation broth | | Filtrate | | |
|---|---|---|---|---|---|---|---|---|---|---|
| c(Ca) | | c(PO$_4$) | | Ratio (%) | pH | PCV (%) | DCW (%) | DCW (%) | Microbial cell removal rate (%) | Turbidity NTU |
| (%, w/w) | mM | (%, w/w) | mM | | | | | | | |
| 0.00 | 0.0 | 0.00 | 0.0 | - | 6.7 | 21.7 | 2.60 | 1.23 | 52.7 | >1000 |

As shown in Table 1 above, it was confirmed that when **$NaH_2PO_4$** and $CaCl_2$ were not added, the microbial cell removal rate was 52.7%, and the turbidity was 1000 NTU or more.

**<Example 1>**

The microbial cell removal rates of $NaH_2PO_4$ and $CaCl_2$ were evaluated by adding 0.5% $CaCl_2$ and 0.8% to 1.0% $NaH_2PO_4$, based on the weight of the *Bacillus subtilis* fermentation broth, and by adjusting pH of the fermentation broth from 6.7 to 7.0 or higher using 10% NaOH solution. The results are shown in Table 2 below.

[Table 2]

| Evaluation conditions | | | | | | Fermentation broth | | Filtrate | | |
|---|---|---|---|---|---|---|---|---|---|---|
| c(Ca) | | c(PO$_4$) | | Ratio (%) | pH | PCV (%) | DCW (%) | DCW (%) | Microbial cell removal rate (%) | Turbidity |
| (%, w/w) | mM | (%, w/w) | mM | | | | | | | NTU |
| 0.00 | 0.0 | 0.00 | 0.0 | - | 6.7 | 21.7 | 2.60 | 1.23 | 52.7 | >1000 |
| 0.5 | 45.1 | 0.8 | 56.4 | 1:16 | 7.0 | 26.7 | 3.40 | 0.33 | 90.3 | 31 |
| | | 0.9 | 63.4 | 1:1.8 | | 27.5 | 2.72 | 0.01 | 99.6 | 10 |
| | | 1.0 | 70.4 | 1: 2.0 | | 26.2 | 2.78 | 0.01 | 99.6 | 7 |

As shown in Table 2 above, it was confirmed that when NaH$_2$PO$_4$ and CaCl$_2$ were added, the microbial cell removal rates were 90.3% to 99.6%. It was also confirmed that the microbial cell removal efficiency was improved by 37.7% to 46.9%, as compared to the control group to which NaH$_2$PO$_4$ and CaCl$_2$ were not added.

**<Example 2>**

The microbial cell removal rates of NaH$_2$PO$_4$ and CaCl$_2$ were evaluated by adding 0.75% CaCl$_2$ and 1.2% to 1.5% NaH$_2$PO$_4$, based on the weight of the *Bacillus subtilis* fermentation broth, and by adjusting pH to 7.0. The results are shown in Table 3 below.

[Table 3]

| Evaluation conditions | | | | | | Fermentation broth | | Filtrate | | |
|---|---|---|---|---|---|---|---|---|---|---|
| c(Ca) | | c(PO$_4$) | | Ratio (%) | pH | PCV (%) | DCW (%) | DCW (%) | Microbial cell removal rate (%) | Turbidity |
| (%, w/w) | mM | (%, w/w) | mM | | | | | | | NTU |
| 0.00 | 0.0 | 0.00 | 0.0 | - | 6.7 | 21.7 | 2.60 | 1.23 | 52.7 | >1000 |
| 0.75 | 67.6 | 1.2 | 84.5 | 1:1.6 | 7.0 | 26.8 | 2.92 | 0.02 | 99.3 | 9 |
| | | 1.35 | 95.1 | 1:1.8 | | 29.2 | 2.95 | 0.02 | 99.3 | 6 |
| | | 1.5 | 105.7 | 1: 2.0 | | 29.6 | 3.09 | 0.03 | 99.0 | 8 |

As shown in Table 3 above, it was confirmed that when NaH$_2$PO$_4$ and CaCl$_2$ were added, the microbial cell removal rates were 99.0% to 99.3%. It was also confirmed that the microbial cell removal efficiency was improved by 46.3% to 46.6%, as compared to the control group to which NaH$_2$PO$_4$ and CaCl$_2$ were not added.

**<Example 3>**

The microbial cell removal rates of NaH$_2$PO$_4$ and CaCl$_2$ were evaluated by adding 1.0% CaCl$_2$ and 1.6% to 2.0% NaH$_2$PO$_4$, based on the weight of the *Bacillus subtilis* fermentation broth, and by adjusting pH to 7.0. The results are shown in Table 4 below.

[Table 4]

| Evaluation conditions | | | | | | Fermentation broth | | Filtrate | | |
| c(Ca) | | c(PO₄) | | Ratio (%) | pH | PCV (%) | DCW (%) | DCW (%) | Microbial cell removal rate (%) | Turbidity |
| (%, w/w) | mM | (%, w/w) | mM | | | | | | | NTU |
|---|---|---|---|---|---|---|---|---|---|---|
| 0.00 | 0.0 | 0.00 | 0.0 | - | 6.7 | 21.7 | 2.60 | 1.23 | 52.7 | >1000 |
| 1.0 | 90.1 | 1.60 | 112.7 | 1 : 1.6 | 7.0 | 30.3 | 3.56 | 0.08 | 97.9 | 15 |
| | | 1.80 | 126.8 | 1:1.8 | | 30.7 | 3.44 | 0.16 | 95.3 | 23 |
| | | 2.00 | 140.9 | 1 : 2.0 | | 31.6 | 3.70 | 0.29 | 92.2 | 40 |

As shown in Table 4 above, it was confirmed that when NaH₂PO₄ and CaCl₂ were added, the microbial cell removal rates were 92.2% to 97.9%. It was also confirmed that the microbial cell removal efficiency was improved by 39.5% to 45.2%, as compared to the control group to which NaH₂PO₄ and CaCl₂ were not added.

**<Example 4>**

The microbial cell removal rates of NaH₂PO₄ and CaCl₂ were evaluated by adding 1.5% CaCl₂ and 2.4% to 3.0% NaH₂PO₄, based on the weight of the *Bacillus subtilis* fermentation broth, and by adjusting pH to 7.0. The results are shown in Table 5 below.

[Table 5]

| Evaluation conditions | | | | | | Fermentation broth | | Filtrate | | |
| c(Ca) | | c(PO₄) | | Ratio (%) | pH | PCV (%) | DCW (%) | DCW (%) | Microbial cell removal rate (%) | Turbidity |
| (%, w/w) | mM | (%, w/w) | mM | | | | | | | NTU |
|---|---|---|---|---|---|---|---|---|---|---|
| 0.00 | 0.0 | 0.00 | 0.0 | - | 6.7 | 21.7 | 2.60 | 1.23 | 52.7 | >1000 |
| 1.5 | 135.2 | 2.40 | 169.1 | 1 : 1.6 | 7.0 | 33.1 | 3.88 | 0.71 | 81.7 | 74 |
| | | 2.70 | 190.2 | 1 : 1.8 | | 33.0 | 4.04 | 0.73 | 81.9 | 75 |
| | | 3.00 | 211.3 | 1 : 2.0 | | 33.8 | 4.08 | 0.75 | 81.5 | 79 |

As shown in Table 5 above, it was confirmed that when NaH₂PO₄ and CaCl₂ were added, the microbial cell removal rates were 81.5% to 81.9%. It was also confirmed that the microbial cell removal efficiency was improved by 28.8% to 29.2%, as compared to the control group to which NaH₂PO₄ and CaCl₂ were not added.

**<Example 5>**

The microbial cell removal rates of NaH₂PO₄ and CaCl₂ were evaluated by adding 2.0% CaCl₂ and 3.2% to 4.0% NaH₂PO₄, based on the weight of the *Bacillus subtilis* fermentation broth, and by adjusting pH to 7.0. The results are shown in Table 6 below.

[Table 6]

| Evaluation conditions | | | | | | Fermentation broth | | Filtrate | | |
| c(Ca) | | c(PO₄) | | Ratio (%) | pH | PCV (%) | DCW (%) | DCW (%) | Microbial cell removal rate (%) | Turbidity |
| (%, w/w) | mM | (%, w/w) | mM | | | | | | | NTU |
|---|---|---|---|---|---|---|---|---|---|---|
| 0.00 | 0.0 | 0.00 | 0.0 | - | 6.7 | 21.7 | 2.60 | 1.23 | 52.7 | >1000 |

(continued)

| Evaluation conditions | | | | | | Fermentation broth | | Filtrate | | |
|---|---|---|---|---|---|---|---|---|---|---|
| 2.0 | 180.2 | 3.20 | 225.4 | 1 : 1.6 | 7.0 | 36.2 | 6.94 | 2.12 | 69.4 | 315 |
| | | 3.60 | 253.6 | 1:1.8 | | 36.6 | 6.96 | 2.11 | 69.6 | 314 |
| | | 4.00 | 281.8 | 1 : 2.0 | | 36.9 | 6.97 | 2.16 | 69.0 | 321 |

As shown in Table 6 above, it was confirmed that when $NaH_2PO_4$ and $CaCl_2$ were added, the microbial cell removal rates were 69.0% to 69.6%. It was also confirmed that the microbial cell removal efficiency was improved by 16.3% to 16.9%, as compared to the control group to which $NaH_2PO_4$ and $CaCl_2$ were not added.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A method of separating and removing *Bacillus subtilis* cells from a *Bacillus subtilis* fermentation broth, the method comprising the steps of:

   preparing the *Bacillus subtilis* fermentation broth;
   adding calcium chloride ($CaCl_2$) and sodium dihydrogen phosphate ($NaH_2PO_4$) to the *Bacillus subtilis* fermentation broth; and
   removing the *Bacillus subtilis* cells from the fermentation broth to which the calcium chloride and sodium dihydrogen phosphate have been added,
   wherein the calcium chloride is comprised in an amount of 0.5 parts by weight to 0.75 parts by weight, based on the total 100 parts by weight of the composition,
   the sodium dihydrogen phosphate is comprised in an amount of 0.9 parts by weight to 1.5 parts by weight, based on the total 100 parts by weight of the composition, and
   the calcium chloride and sodium dihydrogen phosphate are comprised in a mixing ratio of 1 : 1.6 to 1 : 2.

2. The method of claim 1, not comprising the step of diluting the *Bacillus subtilis* fermentation broth.

3. The method of claim 1, wherein the *Bacillus subtilis* fermentation broth is prepared by culturing *Bacillus subtilis* in a medium.

4. The method of claim 1, wherein the fermentation broth from which the *Bacillus subtilis* cells have been removed has a *Bacillus subtilis* cell removal rate of 99% or more.

5. The method of claim 1, further comprising the step of adjusting pH of the *Bacillus subtilis* fermentation broth to 6.5 to 7.5, before the step of removing the *Bacillus subtilis* cells from the fermentation broth to which the calcium chloride and sodium dihydrogen phosphate have been added.

6. A composition for removing *Bacillus subtilis* cells from a *Bacillus subtilis* fermentation broth, the composition comprising calcium chloride and sodium dihydrogen phosphate,

   wherein the calcium chloride is comprised in an amount of 0.5 parts by weight to 0.75 parts by weight, based on the total 100 parts by weight of the composition,
   the sodium dihydrogen phosphate is comprised in an amount of 0.9 parts by weight to 1.5 parts by weight, based on the total 100 parts by weight of the composition, and
   the calcium chloride and sodium dihydrogen phosphate are comprised in a mixing ratio of 1 : 1.6 to 1 : 2.

7. The composition of claim 6, wherein the pH of the composition is 6.5 to 7.5.

[FIG. 1]

```
┌─────────────────────────────────────────┐
│           Fermentation broth             │
└─────────────────────────────────────────┘
                    │
                    ▼
┌─────────────────────────────────────────┐
│         Addition of Ca, PO4 (w/w)        │
└─────────────────────────────────────────┘
                    │
                    ▼
┌─────────────────────────────────────────┐
│         Agitation, sedimentation         │
└─────────────────────────────────────────┘
                    │
                    ▼
┌─────────────────────────────────────────┐
│            Filtration process            │
└─────────────────────────────────────────┘
                    │
                    ▼
┌─────────────────────────────────────────┐
│          Concentration process           │
└─────────────────────────────────────────┘
                    │
                    ▼
┌─────────────────────────────────────────┐
│          Liquid, solid products          │
└─────────────────────────────────────────┘
```

## INTERNATIONAL SEARCH REPORT

International application No.

**PCT/KR2024/008471**

### A. CLASSIFICATION OF SUBJECT MATTER

**C12N 1/02**(2006.01)i; **C12N 1/20**(2006.01)i; C12R 1/125(2006.01)n

According to International Patent Classification (IPC) or to both national classification and IPC

### B. FIELDS SEARCHED

Minimum documentation searched (classification system followed by classification symbols)

C12N 1/02(2006.01); B01D 21/01(2006.01); C12N 1/08(2006.01); C12N 1/20(2006.01); C12Q 1/00(2006.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 바실러스 서브틸리스(Bacillus subtilis), 염화칼슘(calcium chloride, CaCl2), 인산나트륨(sodium phosphate, NaH2PO4), 발효(fermentation), 분리(separation)

### C. DOCUMENTS CONSIDERED TO BE RELEVANT

| Category* | Citation of document, with indication, where appropriate, of the relevant passages | Relevant to claim No. |
|---|---|---|
| A | JP 2001-037468 A (KURITA WATER IND LTD.) 13 February 2001 (2001-02-13)<br>See paragraphs [0005] and [0008]; example 1; and claim 1. | 1-7 |
| A | JP 10-075769 A (KURITA WATER IND LTD.) 24 March 1998 (1998-03-24)<br>See entire document. | 1-7 |
| A | JP 2001-178495 A (KURITA WATER IND LTD.) 03 July 2001 (2001-07-03)<br>See entire document. | 1-7 |
| A | US 2006-0154247 A1 (BAKER, M. J. et al.) 13 July 2006 (2006-07-13)<br>See entire document. | 1-7 |
| A | KR 10-1994-0014767 A (MIWON CO.) 19 July 1994 (1994-07-19)<br>See entire document. | 1-7 |

☐ Further documents are listed in the continuation of Box C.       ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **07 October 2024** | **07 October 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/008471**

| Patent document cited in search report | | | Publication date (day/month/year) | Patent family member(s) | | | Publication date (day/month/year) |
|---|---|---|---|---|---|---|---|
| JP | 2001-037468 | A | 13 February 2001 | None | | | |
| JP | 10-075769 | A | 24 March 1998 | JP | 3216543 | B2 | 09 October 2001 |
| JP | 2001-178495 | A | 03 July 2001 | None | | | |
| US | 2006-0154247 | A1 | 13 July 2006 | AU | 2003-244749 | A1 | 19 December 2003 |
| | | | | CA | 2486616 | A1 | 11 December 2003 |
| | | | | EP | 1509603 | A1 | 02 March 2005 |
| | | | | JP | 2005-531304 | A | 20 October 2005 |
| | | | | WO | 03-102184 | A1 | 11 December 2003 |
| KR | 10-1994-0014767 | A | 19 July 1994 | KR | 10-1996-0006578 | B1 | 20 May 1996 |

Form PCT/ISA/210 (patent family annex) (July 2022)

**REFERENCES CITED IN THE DESCRIPTION**

**Patent documents cited in the description**

- KR 1020130022059 A

- KR 100411238 B1